# EUROPÄISCHE PATENTSCHRIFT

(11) **EP 0 655 905 B1**
(45) Veröffentlichungstag und Bekanntmachung des Hinweises auf die Patenterteilung: **23.10.1996**
(21) Anmeldenummer: 94908186.3
(22) Anmeldetag: 13.08.1993
(51) Int. Cl.: A61K 7/13, A61K 7/50

(54) **ALKYLGLYCOSIDE IN FÄRBEMITTELN**
ALKYL GLYCOSIDES IN DYEING AGENTS
GLYCOSIDES D'ALKYLE DANS DES COLORANTS

(30) Priorität: 22.08.1992 DE 4227864
(43) Veröffentlichungstag der Anmeldung: 07.06.1995
(73) Patentinhaber: Henkel Kommanditgesellschaft auf Aktien, 40191 Düsseldorf (DE)
(72) Erfinder: MATZIK, Iduna, D-40671 Erkrath (DE); HÖFFKES, Horst, D-40595 Düsseldorf (DE); HOLLENBERG, Detlef, D-40699 Erkrath (DE); MÜLLER, Reinhard, D-41812 Erkelenz (DE); EHLERT, Manuela, D-51379 Leverkusen (DE); SCHRAMM, Christa, D-40474 Düsseldorf (DE)
(86) Internationale Anmeldenummer: EP9302160
(87) Internationale Veröffentlichungsnummer: WO9404124

(56) Entgegenhaltungen:
- EP-A- 0 548 620
- DE-A- 4 129 926
- GB-A- 2 186 891

## Beschreibung

Gegenstand der Erfindung sind Mittel zum Färben von keratinhaltigen Fasern auf Basis von Oxidationsfarbstoffen enthaltend ein C₁₂-C₁₆-Alkylglycosid und vorzugsweise zusätzlich ein amphoteres oder zwitterionisches Polymer.

Übliche Mittel zuin Färben von keratinhaltigen Fasern enthalten als farbgebende Komponente Oxidationsfarbstoffvorprodukte, die in Gegenwart von Oxidationsmitteln den eigentlichen Farbstoff ausbilden. Weiterhin enthalten solche Färbemittel Tenside, die das Benetzen der Faser und einen ausreichenden Farbaufzug auf die Faser gewährleisten sollen. Die üblichen den Färbemitteln gute färbetechnische Eigenschaften verleihenden Tenside verfügen nicht immer über befriedigende Hautverträglichkeiten. Es hat daher nicht an Versuchen gefehlt, Färbemittel mit hautverträglichen Tensiden bereitzustellen, die gleichzeitig über ein ausreichendes Schaumvermögen und gute färbetechnische Eigenschaften hinsichtlich z.B. Farbaufzug auf die Faser und Egalisierverhalten verfügen.

So beschreibt die europäische Offenlegungsschrift EP-462 857 ein Haarfärbemittel auf Basis eines Oxidationsfarbstoffs, das ein Fettalkoholethersulfat und ein C₈-C₁₀-Alkylglycosid enthält.

Die europäische Patentschrift EP 070 074 offenbart schäumende Zusammensetzungen enthaltend Alkylpolyglycosid und ein Aniontensid, die fakultativ Farbstoffe enthalten können; der Schrift ist jedoch kein Hinweis auf ein Färbemittel für Keratinfasern zu entnehmen.

Die deutsche Patentschrift DE 41 29 926 beschreibt Haartönungsshampoos auf Basis von direktziehenden Farbstoffen enthaltend ein C₉-C₁₁-Alkylpolyglycosid, ein Aniontensid und ein Aminoxid.

Die europäische Offenlegungsschrift EP-A1-0 548 620, welche Stand der Technik gemäß Art. 54(3) EPÜ ist, offenbart Haarfärbemittel mit Oxidationsfarbstoffvorprodukten und bestimmten Enzymen, die gemäß einer bevorzugten Ausführungsform nichtionische Tenside vom Typ der Alkylglycoside enthalten können. In einem Beispiel wird ein Mittel offenbart, das unter anderem C₉₋₁₁-Alkylpolyglycosid enthält.

Es wurde nun gefunden, daß Färbemittel auf Oxidationsfarbstoffbasis enthaltend ein C₁₂-C₁₆-Alkylglycosid sowohl über hervorragende Hautverträglichkeit als auch über sehr gute färbetechnische Eigenschaften wie z. B. Farbaufzug auf die Faser und Egalisierung verfügen. Weiterhin weisen die damit erzielten Färbungen sehr gute Echtheitseigenschaften auf.

Gegenstand der Erfindung sind Mittel zum Färben von keratinhaltigen Fasern enthaltend
- ein Alkylglycosid der Formel (I)

   R¹ - O - [G]ₓ - H (I),

   wobei R¹ für einen linearen oder verzweigten Alkyl- oder Alkenylrest mit 12 bis 16 C-Atomen, [G] für einen Anhydroglucoserest und x für eine Zahl von 1 bis 2,5, vorzugsweise 1,1 bis 1,6 steht,
- ein Oxidationsfarbstoffvorprodukt und
- einen wäßrigen Träger.

Keratinhaltige Fasern sind z. B. menschliche und tierische Haare, Pelze, Wolle oder Federn.

Oxidationsfarbstoffvorprodukte sind Farbstoffvorläufer, die unter dem Einfluß von Oxidationsmitteln oder von Luftsauerstoff im Zuge einer oxidativen Polymerisation die eigentlichen Farbstoffe ausbilden. Neben Oxidationsfarbstoffvorprodukten vom Entwickler-Typ verwendet man vorteilhafterweise zusätzlich solche vom Kuppler-Typ, die während der Polymerisation mit den Vorprodukten vom Entwicklertyp kuppeln und mit deren Hilfe so die gewünschten Farbnuancen eingestellt werden können. Übliche Vorprodukte vom Entwickler-Typ sind primäre aromatische Amine mit einer weiteren in Para- oder Ortho-Position befindlichen freien oder substituierten Hydroxy- oder Aminogruppe, ferner Diaminopyridinderivate, heterocyclische Hydrazonderivate, 4-Aminopyrazolonderivate, Tetraaminopyrimidinderivate und Indol- und Indolinderivate; übliche Vorprodukte vom Kuppler-Typ sind z.B. Metaphenylendiaminderivate, Dihydroxynaphthaline, Naphthole, Resorcinderivate, Metaaminophenole und Pyrazolone.

Der wäßrige Träger ist im einfachsten Falle Wasser oder eine wäßrig/alkoholische Mischung, vorzugsweise aber eine Emulsion, ein Gel oder eine tensidhaltige schäumende Lösung.

Die in den erfindungsgemäßen Färbemitteln enthaltenen C₁₂-C₁₆-Alkylglycoside sind den C₈-C₁₁-Alkylglycosiden hinsichtlich des Farbaufzugs auf die Faser deutlich überlegen. Die farbverstärkende Wirkung und die damit verbundene Farbstoffeinsparung beim Färben erreicht unerwarteter Weise bei den C₁₂-C₁₆-Alkylglycosiden ein Maximum, um dann mit den C₁₈-alkylsubstituierten Glycosiden beginnend wieder abzufallen.

Im Hinblick auf das Schaumverhalten der erfindungsgemäßen Färbemittel lassen sich die C₁₂-C₁₆-Alkylglycoside in idealer Weise mit weiteren Tensiden kombinieren. Als weitere Tenside kommen anionische, kationische, nichtionische, amphotere und zwitterionische Tenside in Frage.

Anionische Tenside sind z.B. Fettalkylethercarboxylate, Sulfosuccinate, Ölsäuresulfonate, Fettalkylethercitrate und Tartrate, Fettacylglutamate, Fettacylisethionate, Fettacyltauride, Fettacylsarkoside, α-Sulfofettsäuren und deren Salze, Sorbitanestersulfate, Hydroxyalkylsorbitolcitrate, Seifen, Fettalkylsulfate, Alkansulfonate und α-Olefinsulfonate.

Kationische Tenside sind z.B. C₁₂-C₁₈-Alkyltrimethylammoniumsalze, C₁₂-C₁₈-Alkyldimethylbenzylammoniumsalze, Cetylpyridiniumchlorid, 2-Hydroxydodecyl-hydroxyethyl-dimethylammoniumchlorid C₁₂-C₁₈-Dialkyldimethylammoniumsalze und C₁₂-C₁₈-Trialkylmethylammoniumsalze.

Nichtionische Tenside sind z.B. Anlagerungsprodukte von 5 - 30 Mol Ethylenoxid an Fettalkohole, an Alkylphenole, an Fettsäuren, an Fettsäurealkanolamide, an Fettsäurepartialglyceride, an Fettsäuresorbitanpartialester oder an Fettsäure-methylglucosid-partialester, ferner Fettsäure-polyglycerinester und Fettacylglucosamine.

Amphotere Tenside sind z.B. N-Dodecylaminoessigsäure, N-Cetylaminopropionsäure, gamma-Laurylamino-buttersäure.

Zwitterionische Tenside sind z.B. C₁₂-C₁₈-Alkyl-dimethyl-ammoniumglycinat, Kokosacylaminopropyl-dimethyl-ammonium-glycinat oder Imidazoliniumbetaine.

Wegen möglicher Verunreinigungen durch Nitrosamine wird in den erfindungsgemäßen Färbemitteln vorzugsweise auf den Einsatz von Aminoxiden verzichtet.

In den erfindungsgemäßen Färbemitteln sind die Alkylglycoside der Formel (I) hinsichtlich färbetechnischer Eigenschaften schon in geringen Mengen wirksam. Ein weiterer Erfindungsgegenstand sind deshalb Mittel zum Färben von keratinhaltigen Fasern, wobei das Alkylglycosid der Formel (I) in einer Menge von 0,05 bis 5 Gew.-%, vorzugsweise 0,1 bis 2,4 Gew.-%, bezogen auf die gesamte Färbemittel-Zubereitung, enthalten ist.

Besonders gute Ergebnisse, vor allem hinsichtlich der Naßkämmbarkeit der gefärbten Fasern und der Waschechtheit der Färbungen werden erzielt, wenn man dem Färbemittel ein amphoteres oder zwitterionisches Polymer zugibt.

Ein weiterer Erfindungsgegenstand sind deshalb Mittel zum Färben von keratinhaltigen Fasern, die zusätzlich ein amphoteres oder zwitterionisches Polymer in einer Menge von 0,02 bis 5 Gew.-%, vorzugsweise 0,1 bis 2 Gew.-%, bezogen auf die gesamte Färbemittel-Zubereitung, enthalten.

Als amphotere Polymere kommen, nicht einschränkend zu verstehen, z.B. in Frage: amphotere Celluloseester oder Copolymerisate aus anionischen und kationischen Vinylmonomeren; ganz besonders eignen sich die zwitterionischen Polymerisate, die durch Quarternierung der Reaktionsprodukte aus Acrylamiden, wie z.B. Dimethylaminoethylacrylamid, Dimethylaminoethylmethacrylamid, Dimethylaminopropylacrylamid, Dimethylaminopropylmethacrylamid, Diethylaminoethylacrylamid, Dimethylaminoethylacrylat, Dimethylaminoethylmethacrylat oder Diethylaminoethylacrylat, und Acrylsäure bzw. substituierten Acrylsäuren, wie z.B. Methacrylsäure, Crotonsäure oder 2-Methylcrotonsäure gebildet werden.

Es ist dabei nicht erforderlich, daß ein einheitliches Polymer verwendet wird, vielmehr können auch Gemische von verschiedenen amphoteren oder zwitterionischen Polymeren zum Einsatz kommen.

Fakultativ können die erfindungsgemäßen Färbemittel auch übliche Kationpolymere wie z.B. quaternisierte Celluloseether, Dimethyldiallylammoniumhomopolymere, Dimethyldiallylammonium/Acrylsäure- und Acrylamidcopolymere, quaternisierte Vinylpyrrolidon/Dimethylaminoethylacrylatcopolymere und Copolymerisate aus Vinylimidazoliniummethochlorid und Vinylpyrrolidon enthalten.

Die erfindungsgemäßen Färbemittel ergeben bereits bei physiologisch verträglichen Temperaturen unterhalb 40°C intensive Färbungen. Ganz besonders eignen sich deshalb die Färbemittel zum Färben von menschlichen Haaren.

Ein weiterer Erfindungsgegenstand sind Haarfärbemittel enthaltend
- ein Alkylglycosid der Formel (I) in einer Menge von 0,05 bis 5 Gew.-%, vorzugsweise 0,1 bis 2,4 Gew.-%,
- ein Oxidationsfarbstoffvorprodukt in einer Menge von 0,01 bis 5 Gew.-%,
- ein amphoteres oder zwitterionisches Polymer in einer Menge von 0,02 bis 5 Gew.-%, vorzugsweise 0,1 bis 2 Gew.-%, jeweils bezogen auf die gesamte Farbemittel-Zubereitung, und
- einen wäßrigen Träger.

Der wäßrige Träger ist vorzugsweise eine Creme, eine Emulsion, ein Gel, eine tensidhaltige, schäumende Lösung oder eine andere Zubereitung, die für die Anwendung auf dem Haar geeignet ist.

Übliche Bestandteile solcher Träger sind Konfektionierungs- und Färbehilfsmittel, welche die Stabilität der Zubereitungen erhöhen und das Ergebnis der Färbung verbessern. Solche Zusätze sind z.B.:
- wasserlösliche, verdickende Polymere (Hydrokolloide), z.B. Celluloseether wie Carboxymethylcellulose, Hydroxyethylcellulose, Methylcellulose, Methyl-hydroxypropylcellulose, Stärke und Stärkeether, Pflanzengumme, Guar-Gum, Agar-Agar, Alginate, Xanthan-Gum oder synthetische wasserlösliche Polymere,
- Öl- und Fettsubstsanzen im weitesten Sinne, z.B. Paraffinöl, Fettsäureester, Fettalkohole, auch z.B. verzweigte Alkohole wie 2-Octyldodecanol-1 oder 2-Hexyldecanol-1,
- Antioxidantien, z.B. Ascorbinsäure, Natriumsulfit,
- Puffersubstanzen, z.B. Ammoniumchlorid und Ammoniumsulfate,
- Komplexbildner, z.B. 1-Hydroxyethan-1,1-diphosphonsäure, Nitrilotriessigsäure oder Ethylendiamintetraessigsäure oder deren Salze,
- haarkosmetische Hilfsmittel, z.B. Proteinderivate, Glucose, D-Panthenol, Cholesterin, Vitamine oder Pflanzenextrakte,
- Egalisierhilfsmittel, z.B. Urazol, Hexahydropyrimidin-2-on, Imidazol, 1,2,4-Triazol oder Jodide, z.B. Natrium- oder Kaliumjodid

Die Anwendung der Haarfarbemittel kann unabhängig von der Art der kosmetischen Zubereitung. z.B. als Creme, Gel oder Shampoo, im schwach sauren, neutralen oder alkalischen Milieu erfolgen. Bevorzugt ist die Anwendung der Haarfärbemittel in einem pH-Bereich von 6 bis 10.

In den Haarfärbemitteln können zur Farbnuancierung zusätzlich zu den zwingend erforderlichen Oxidationsfarbstoffvorprodukten fakultativ übliche direktziehende Farbstoffe enthalten sein.

Die erfindungsgemäßen Haarfärbemittel ergeben einen feinen, cremigen Schaum. Sie sind toxikologisch günstig zu bewerten und sehr gut hautverträglich. Das Optimum der Hautverträglichkeit wird erzielt, wenn die patentgemäß beanspruchten C₁₂-C₁₆-Alkylglycoside verwendet werden; sie sind sowohl den kürzerkettigen C₈-C₁₁-Alkylglycosiden als auch den längerkettigen C₁₈-C₂₂-Alkylglycosiden überlegen.

Färbetechnische Eigenschaften wie Farbaufziehvermögen und Egalisierverhalten sind hervorragend. Die erzielten Färbungen zeichnen sich durch brillante Farben und hohe Waschechtheit aus. Die gefärbten Haare verfügen über eine sehr gute Naßkämmbarkeit.

Die folgenden Beispiele sollen den Erfindungsgegenstand erläutern, ohne ihn jedoch hierauf zu beschränken.

### Beispiele

Es wurde eine Haarfärbecreme-Basis der folgenden Zusammensetzung hergestellt (Tabelle I)

**Tabelle I**

| | |
|---|---|
| Talgfettalkohol | 6,5 g |
| Kokosfettalkohol | 2,0 g |
| p-Toluylendiamin (Entwickler) | 0,52 g |
| Resorcin (Kuppler) | 0,10 g |
| 4-Chlorresorcin (Kuppler) | 0,15 g |
| 3-Amino-2-methylamino-6-methoxy-pyridin (Entwickler) | 0,03 g |
| Ammoniumsulfat | 1,4 g |
| Natriumsulfit (Inhibitor) | 0,5 g |
| Ascorbinsäure (Inhibitor) | 0,4 g |
| Paraffinöl | 0,2 g |
| Ammoniak (30 %ig) | 5,7 g |

Die Bestandteile wurden der Reihe nach miteinander vermischt. Nach Zugabe der in den folgenden Beispielen 1 bis 9 aufgelisteten zusätzlichen Komponenten, der Entwicklersubstanzen und der Inhibitoren wurde zunächst mit der Ammoniak-Lösung ein pH-Wert von 9,5 eingestellt, dann wurde mit Wasser auf 100 g aufgefüllt.

### Beispiele 1 bis 9 (Tabelle II)

### (Die Beispiele 4 bis 9 sind erfindungsgemäß)

| | **1** | **2** | **3** | **4** | **5** |
|---|---|---|---|---|---|
| C_{12/16}-Fettalkoholglycosid (Plantaren 1200)* | - | - | - | 2,4 g | 1,0 g |
| C_{12/14}-Fettalkohol-4,5 EO-essigsäure, Ammonium-Salz (Akypo RLM 45 A) ** | - | 4,9 g | 3,5 g | 2,5 g | 2,9 g |
| Na-laurylethersulfat (Texapon N 25)* | 4,9 g | - | 1,4 g | - | 1,0 g |

| | **6** | | **7** | **8** | **9** |
|---|---|---|---|---|---|
| C_{12/16}-Fettalkoholglycosid (Plantaren 1200)* | 1,0 g | | 1,0 g | 1,0 g | 2,4 g |
| C_{12/14}-Fettalkohol-4,5 EO-essigsäure, Ammonium-Salz (Akypo RLM 45A) ** | 2,9 g | | 1,9 g | - | 2,5 g |
| Na-laurylethersulfat (Texapon N 25)* | 1,0 g | | 2,0 g | 3,9 g | - |
| Acrylsäure, Na-Salz/Acrylamidopropyltrimethylammoniumchlorid-Copolymer (Polymer P1 gemäß DE-OS 39 29 973) | 0,3 g | | 0,3 g | 0,6 g | 0,6 g |

| | | | | | |
|---|---|---|---|---|---|
| * Firma Henkel KGaA | | | | | |
| ** Firma Chem-Y | | | | | |

Die oxidative Entwicklung der Färbung wurde mit 6 %iger Wasserstoffperoxidlösung als Oxidationslösung durchgeführt. Hierzu wurden 50 g der Emulsion mit 50 g Wasserstoffperoxidlösung (6 %ig) versetzt und vermischt.

Die Färbecreme wurde auf ca. 5 cm lange Strähnen standardisierten, zu 90 % ergrauten - aber nicht besonders vorbehandelten - Menschenhaar aufgetragen und dort 30 Minuten bei 27°C belassen. Nach Beendigung des Färbeprozesses wurde das Haar gespült, mit einem üblichen Haarwaschmittel gewaschen und anschließend getrocknet.

Die mit den Zusammensetzungen 1 bis 9 erzielte Farbnuance war hellbraun.

In gleicher Weise wurden Egalisiersträhnen gefärbt; Egalisiersträhnen wurden wie folgt hergestellt:
Die obere Hälfte der Haarsträhne (der Bereich der Haarspitze) wurde 30 Minuten lang mit der wäßrigen Lösung eines Kaltwellmittels auf Basis Ammoniumthioglykolat behandelt. Nach der Fixierung (10 Minuten, Kaliumbromat-Lösung) wurde die gleiche Hälfte der Haarsträhne mit einer wäßrigen Lösung aus Wasserstoffperoxid und Ammoniumperoxiddisulfat ultrablondiert. Anschließend erfolgte noch einmal eine Behandlung mit dem Kaltwellmittel, der Fixierung und der Ultrablondierung. Die untere Hälfte der Haarsträhne (Wurzelbereich) wurde nur einmal ultrablondiert. Auf diese Weise wurden 2 unterschiedlich stark strapazierte Haarsträhnen-Hälften erhalten.

In Tabelle III sind die Ergebnisse hinsichtlich Farbaufzug, Waschechtheit und Egalisierverhalten der Zusammensetzungen 1 bis 9 aufgeführt.

Die Egalisierung wird in DE-Werten (Gesamt-Farbabstand zwischen den beiden Enden der Egalisiersträhne) angegeben. Die DE-Werte wurden wie folgt ermittelt:

Jede Egalisier-Haarsträhne wurde an acht Stellen (4 im Bereich des Haaransatzes und 4 im Bereich der Haarspitze) mit Hilfe eines Farbmeßsystems der Firma Datacolor vermessen. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner (IBM - PS 2) verarbeitet. Das Rechnerprogramm ermittelte die Normfarbwerte nach dem CIE-System (Commission Internationale de l'Eclairage) entsprechend DIN 5033 und rechnete sie in Farbabstandszahlen nach DIN 6174 µm.

Als Standard bezüglich Farbaufzug und Waschechtheit diente Zusammensetzung 1. Dazu wurde die Farbintensität der mit Zusammensetzung 1 gefärbten, ungewaschenen Strähne farbmetrisch bestimmt und zu 100 % gesetzt. Die dazu relativen Farbintensitäten der mit den übrigen Zusammensetzungen 2 bis 9 gefärbten, ungewaschenen Haarsträhnen sind Tabelle III, Spalte "Farbaufzug" zu entnehmen.

In gleicher Weise wurde die farbmetrisch bestimmte Farbintensität der mit Zusammensetzung 1 gefärbten, 6 mal gewaschenen Haarsträhne zu 100 % gesetzt. Die dazu relativen Farbintensitäten der mit den Zusammensetzungen 2 bis 9 gefärbten Strähnen sind Tabelle III, Spalte "Waschechtheit" zu entnehmen.

(Anm: Vergleiche hinsichtlich der relativen Farbintensitäten sind somit lediglich innerhalb einer Spalte zulässig.)

**Tabelle III**

| | Rel. Farbintensität der ungewaschenen Strähne (Farbaufzug) | Rel. Farbintensität der 6 mal gewaschenen Strähne (Waschechtheit) | DE-Wert der 6 mal gewaschenen Strähne (Egalisierung) |
|---|---|---|---|
| 1 | 100 % | 100 % | 10,45 |
| 2 | 105 % | 98 % | 9,87 |
| 3 | 106 % | 101 % | 10,19 |
| 4 | 104 % | 99 % | 8,6 |
| 5 | 110 % | 105 % | 8,5 |
| 6 | 112 % | 110 % | 8,7 |
| 7 | 110 % | 105 % | 8,8 |
| 8 | 103 % | 101 % | 9,85 |
| 9 | 106 % | 104 % | 8,87 |

Die erfindungsgemäßen Färbemittel-Zusammensetzungen 4 bis 9 zeigen hinsichtlich Farbaufzug, Egalisierung und Waschechtheit im Durchschnitt deutlich bessere Ergebnisse als die nicht erfindungsgemäßen Zusammensetzungen 1 bis 3.

Auch hinsichtlich Naßkammbarkeit, Griff und Glanz ist eine deutliche Verbesserung gegenüber den nicht erfindungsgemäßen Zusammensetzungen zu beobachten.

In einem weiteren Versuch wurde in den Beispielen 1 bis 9 der Kokosfettalkohol durch 2-Octyldodecanol-1 ersetzt; es zeigte sich, daß die 2-Octyldodecanol-1-haltigen Creme-Zubereitungen über eine erhöhte Kältestabilität verfügen.

Der Ersatz von jeweils 1,0 g Kokosfettalkohol und Talgfettalkohol durch 2,0 g 2-Octyldodecanol-1 in den Beispielen 1 bis 9 führte ebenfalls zu diesem Ergebnis.

## Patentansprüche

1. Mittel zum Färben von keratinhaltigen Fasern enthaltend
- ein Alkylglycosid der Formel (I),
R¹ - O - [ G ]ₓ - H (I),
wobei R¹ für einen linearen oder verzweigten Alkyl- oder Alkenylrest mit 12 bis 16 C-Atomen, [ G ] für einen Anhydroglucoserest und x für eine Zahl von 1 bis 2,5, vorzugsweise 1,1 bis 1,6, steht,
- ein Oxidationsfarbstoffvorprodukt und
- einen wäßrigen Träger.

2. Mittel nach Anspruch 1, dadurch gekennzeichnet, daß das Alkylglycosid der Formel I in einer Menge von 0,05 bis 5 Gew.-%, vorzugsweise 0,1 bis 2,4 Gew.-%, bezogen auf die gesamte Färbemittelzubereitung, enthalten ist.

3. Mittel nach Anspruch 1 und 2, dadurch gekennzeichnet, daß zusätzlich ein amphoteres oder zwitterionisches Polymer in einer Menge von 0,02 bis 5 Gew.-%, vorzugsweise 0,1 bis 2 Gew.-%, bezogen auf die gesamte Färbemittelzubereitung, enthalten ist.

4. Haarfärbemittel enthaltend
- ein Alkylglycosid der Formel (I) in einer Menge von 0,05 bis 5 Gew.-%, vorzugsweise 0,1 bis 2,4 Gew.-%,
- ein Oxidationsfarbstoffvorprodukt in einer Menge von 0,01 bis 5 Gew.-%,
- ein amphoteres oder zwitterionisches Polymer in einer Menge von 0,02 bis 5 Gew.-%, vorzugsweise 0,1 bis 2 Gew.-%, jeweils bezogen auf die gesamte Färbemittelzubereitung und
- einen wäßrigen Träger.

## Claims

1. Preparations for coloring keratin-containing fibers containing
- an alkyl glycoside corresponding to formula (I):
R¹ - O - [G]ₓ - H (I)
in which R¹ is a linear or branched alkyl or alkenyl radical containing 12 to 16 carbon atoms, [G] is an anhydroglucose unit and x is a number of 1 to 2.5 and preferably 1.1 to 1.6,
- an oxidation dye precursor and
- a water-based carrier.

2. Preparations as claimed in claim 1, characterized in that the alkyl glycoside corresponding to formula I is present in a quantity of 0.05 to 5% by weight and preferably in a quantity of 0.1 to 2.4% by weight, based on the coloring preparation as a whole.

3. Preparations as claimed in claims 1 and 2, characterized in that an amphoteric or zwitterionic polymer is additionally present in a quantity of 0.02 to 5% by weight and preferably in a quantity of 0.1 to 2% by weight, based on the hair coloring preparation as a whole.

4. Hair coloring preparations containing
- an alkyl glycoside corresponding to formula (I) in a quantity of 0.05 to 5% by weight and preferably in a quantity of 0.1 to 2.4% by weight,
- an oxidation dye precursor in a quantity of 0.01 to 5% by weight,
- an amphoteric or zwitterionic polymer in a quantity of 0.02 to 5% by weight and preferably in a quantity of 0.1 to 2% by weight, based on the coloring preparation as a whole, and
- a water-based carrier.

## Revendications

1. Agent de teinture de fibres contenant de la kératine comprenant :
- un alkylglycoside de formule (I)
R¹ - 0 - [ G ]ₓ - H (I)
où R¹ représente un radical alkyle ou alcényle linéaire ou ramifié de 12 à 16 atomes de C, [G] un radical anhydroglucose et x un nombre de 1 à 2,5, de préférence 1,1 à 1,6,
- un précurseur de colorant d'oxydation et,
- un véhicule aqueux.

2. Agent selon la revendication 1,
caractérisé en ce que
l'alkylglycoside de formule I est compris en une quantité de 0,05 à 5 % en poids, de préférence 0,1 à 2,4 % en poids, par rapport à la préparation totale de teinture.

3. Agent selon les revendications 1 et 2,
caractérisé en ce que
en plus il comprend un polymère amphotère ou zwitterionique en une quantité de 0,02 à 5 % en poids, de préférence 0,1 à 2 % en poids, par rapport à la préparation totale de teinture.

4. Teinture capillaire contenant :
- un alkylglycoside de formule (I) en une quantité de 0,05 à 5 % en poids, de préférence 0,1 à 2,4 % en poids,
- un précurseur de colorant d'oxydation en une quantité de 0,01 à 5 % en poids,
- un polymère amphotère ou zwitterionique en une quantité de 0,02 à 5 % en poids, de préférence 0,1 à 2 % en poids par rapport à la préparation totale de teinture, respectivement et,
- un véhicule aqueux.
